# EUROPEAN PATENT APPLICATION

(11) **EP 4 485 384 A1**
(43) Date of publication of application: **01.01.2025**
(21) Application number: 24185774.7
(22) Date of filing: 01.07.2024
(51) Int. Cl.: G06V 20/40, G06F 3/01, A61B 5/0205, G06F 3/0481, G06V 40/20, H04N 7/14, H04N 7/15

(54) **EYE TRACKING, PHYSIOLOGY, FACIAL EXPRESSION, AND POSTURE TO MODULATE EXPRESSION**

(30) Priority: 29.06.2023 US 202318216236
(71) Applicant: Rockwell Collins, Inc., Cedar Rapids, IA 52498 (US)
(72) Inventor: WU, Peggy, Ellicott City, MD, 21042 (US)
(74) Representative: Dehns

(57) **Abstract**

A team monitoring system receives video data for determining emotional states and social cues for each team member with respect to a baseline profile of the user. The emotional states and social cues are included in corresponding video streams for each team member. The baseline profile may define idiosyncrasies of the user that can be normalized for relation to the rest of the team to facilitate team interactions. Video streams are modified to include or enhance social clues that may otherwise be lost due to the disposition of cameras and other limitations of teleconferencing.

## Description

### GOVERNMENT LICENSE RIGHTS

The U.S. Government has a paid-up license in this invention and the right in limited circumstances to require the patent owner to license others on reasonable terms as provided by the terms of DE-AR0001097 awarded by The United States Department of Energy.

### BACKGROUND

When working with remote, geographically separated human teams, it can be difficult to assess the group's expressions due to the limited access to social cues. Even with video conferencing, team members have a reduced view of each other compared to in-person interactions, the video quality may be limited or out of focus, and the display may have a small footprint.

### SUMMARY

In one aspect, there is provided a team monitoring system that receives video data for determining emotional states and social cues for each team member with respect to a baseline profile of the user. The emotional states and social cues are included in corresponding video streams for each team member.

In an embodiment, the baseline profile may define idiosyncrasies of the user that can be normalized for relation to the rest of the team to facilitate team interactions.

In an embodiment, video streams are modified to include or enhance social clues that may otherwise be lost due to the disposition of cameras and other limitations of teleconferencing.

It is to be understood that both the foregoing general description and the following detailed description are exemplary and explanatory only and should not restrict the scope of the claims. The accompanying drawings, which are incorporated in and constitute a part of the specification, illustrate exemplary embodiments of the inventive concepts disclosed herein and together with the general description, serve to explain the principles.

### BRIEF DESCRIPTION OF THE DRAWINGS

The numerous advantages of the embodiments of the inventive concepts disclosed herein may be better understood by those skilled in the art by reference to the accompanying figures in which:
- FIG. 1: shows a block diagram of a system suitable for implementing embodiments of the incentive concepts disclosed herein;
- FIG. 2: shows a flowchart of an exemplary embodiment of the inventive concepts disclosed herein;
- FIG. 3: shows a block diagram of a neural network according an exemplary embodiment of the inventive concepts disclosed herein.

### DETAILED DESCRIPTION

Before explaining various embodiments of the inventive concepts disclosed herein in detail, it is to be understood that the inventive concepts are not limited in their application to the arrangement of the components or steps or methodologies set forth in the following description or illustrated in the drawings. In the following detailed description of embodiments of the instant inventive concepts, numerous specific details are set forth in order to provide a more thorough understanding of the inventive concepts. However, it will be apparent to one of ordinary skill in the art having the benefit of the instant disclosure that the inventive concepts disclosed herein may be practiced without these specific details. In other instances, well-known features may not be described in detail to avoid unnecessarily complicating the instant disclosure. The inventive concepts disclosed herein are capable of other embodiments or of being practiced or carried out in various ways. Also, it is to be understood that the phraseology and terminology employed herein is for the purpose of description and should not be regarded as limiting. The scope of the invention is as defined by the claims

**As** used herein a letter following a reference numeral is intended to reference an embodiment of a feature or element that may be similar, but not necessarily identical, to a previously described element or feature bearing the same reference numeral (e.g., 1, 1a, 1b). Such shorthand notations are used for purposes of convenience only, and should not be construed to limit the inventive concepts disclosed herein in any way unless expressly stated to the contrary.

Further, unless expressly stated to the contrary, "or" refers to an inclusive or and not to an exclusive or. For example, a condition A or B is satisfied by anyone of the following: A is true (or present) and B is false (or not present), A is false (or not present) and B is true (or present), and both A and B are true (or present).

In addition, use of "a" or "an" are employed to describe elements and components of embodiments of the instant inventive concepts. This is done merely for convenience and to give a general sense of the inventive concepts, and "a" and "an" are intended to include one or at least one and the singular also includes the plural unless it is obvious that it is meant otherwise.

Also, while various components may be depicted as being connected directly, direct connection is not a requirement. Components may be in data communication with intervening components that are not illustrated or described.

Finally, as used herein any reference to "one embodiment," or "some embodiments" means that a particular element, feature, structure, or characteristic described in connection with the embodiment is included in at least one embodiment of the inventive concepts disclosed herein. The appearances of the phrase "in at least one embodiment" in the specification does not necessarily refer to the same embodiment. Embodiments of the inventive concepts disclosed may include one or more of the features expressly described or inherently present herein, or any combination or sub-combination of two or more such features.

Broadly, embodiments of the inventive concepts disclosed herein are directed to a team monitoring system that receives video data for determining emotional states and social cues for each team member with respect to a baseline profile of the user. The emotional states and social cues are included in corresponding video streams for each team member. The baseline profile may define idiosyncrasies of the user that can be normalized for relation to the rest of the team to facilitate team interactions. Video streams are modified to include or enhance social clues that may otherwise be lost due to the disposition of cameras and other limitations of teleconferencing.

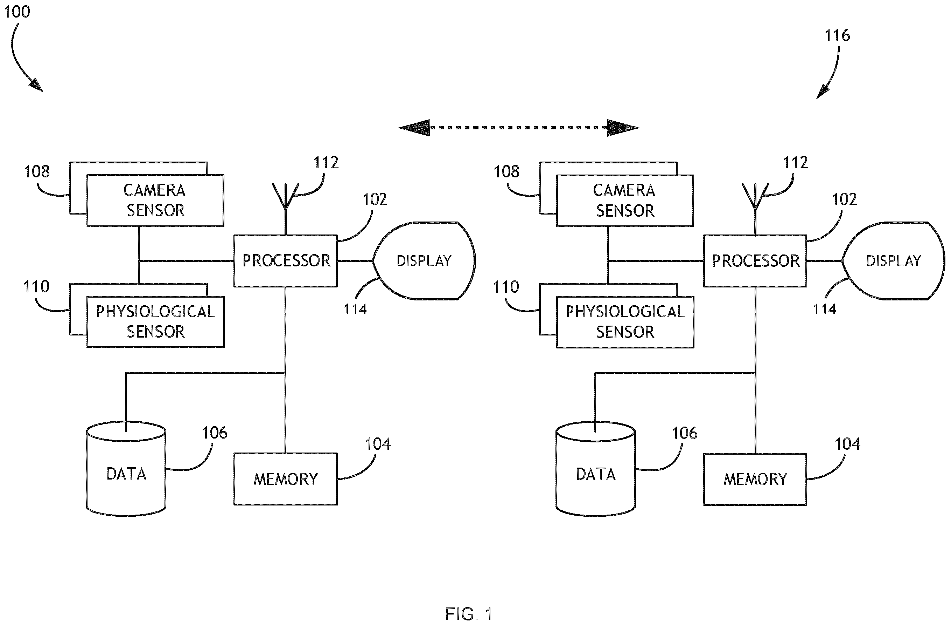

Referring to FIG. 1, a block diagram of a system suitable for implementing embodiments of the incentive concepts disclosed herein is shown. The system includes at least two nodes 100, 116, each including a processor 102, memory 104 in data communication with the processor 102 for storing processor executable code, one or more cameras 108 for receiving a video data stream, and one or more physiological sensors 110. Physiological sensors 110 may include devices such as an electroencephalograph (EEG), functional near-infrared spectroscopy (fNIRs), heart rate monitor, galvanic skin response sensor or any other such biometric data sensing device.

In at least one embodiment, the one or more cameras 108 record eye movement / gaze of a user, eye lid position, hand / arm position and movement, and other physical data landmarks. The processor executable code configures the processor 102 to continuously log the camera data in a data storage element 106. The processor 102 analyzes the camera data to identify gaze and pupil dynamics (e.g., pupil response and changes over time), and identify social cues that may be embodied in facial expressions and the like. Each processor 102 may also receive physiological data from one or more corresponding physiological sensors 110.

In at least one embodiment, the processor 102 may correlate camera data (including at least gaze and pupil dynamics) with physiological data. The processor 102 may compare the camera and physiological data to stored individual profiles, specific to the user. The profiles define individual base line measurements of physiological metrics, gaze, and pupil dynamics with respect to social cues and idiosyncratic expressions specific to the user; comparing instantaneous measurements to the individual baseline measurements may provide a measure of emotions and social cues that may be useful to other team members to gauge the state of the user. The processor 102 may display the measure of emotions and social cues on a display device 114.

The system operates across multiple nodes 100, 116, with each node 100, 116 directed toward an individual team member. Each node 100, 116 may be configured to receive emotional states and social cues for each other connected team member via a data communication device 112. In at least one embodiment, each node 100, 116 may correlate emotional states and social cues for each team member, within the context of discreet portions of a task being performed by the team.

In at least one embodiment, the camera data are correlated with discreet portions of a task, and / or specific stimuli such as instrument readings, alerts, or the like. Furthermore, the processors 102 from each node 100, 116 correlate camera data from different users engaged in a common or collective task. Each processor 102 may receive different discreet portions of a task, specific stimuli, and alerts based on the specific function of the user; such different discreet portions, stimuli, and alerts are correlated in time such that user responses may be individually analyzed and correlated to each other to assess emotional states and social cues.

In at least one embodiment, each node 100, 116 may share camera data between nodes 100, 116 via the data communication device 112 to render on a corresponding display 114 such as in a teleconferencing application. The first team members emotional states and social cues may be characterized with respect to the second team members camera data (facial expression, gaze, pupil dynamics, etc.). The emotional states and social cues of the first team member may be at least partially characterized with respect to the emotional states and social cues of the second team member. For example, the first node may identify a gaze of the first team member and determine that the first team member is observing the second team member; the second team members emotional states and social cues may be associated with a predicted or characteristic response of the first team member. The emotional states and social cues of the first team member may be assessed with respect to the speed and appropriateness of their response to the second team member, including interacting with the second team member.

In at least one embodiment, the processor 102 may apply a modification to the camera data stream based on a team members emotional states and social cues. For example, the first node 100 may identify certain social cues from a first team member with respect to the other team members such as the first team member's gaze focused on a portion of the display 114 corresponding to a second team member. The first node 100 may render individual team members' camera data on the display 114 and determine the placement of each camera data stream on the display 114 corresponding to a particular team member. When another team member is talking, the first node 100 may determine that the gaze of the first team member is focused on the portion of the display 114 including the rendered camera data of the talking team member.

It may be appreciated that gaze is a social cue when individuals are speaking, however, teleconferencing applications often utilize cameras that provide a distorted impression of the user because of their disposition (above the display 114, for example). Each team member node 100, 116 may modify the camera data streams to render team members' pupils to direct the gaze of each team member toward a talking team member based on the disposition of each camera data stream on the corresponding display 114.

In at least one embodiment, the processor 102 may modify camera data streams (incoming or outgoing) to enhance or diminish social cues such as facial expressions. The processor 102 may identify certain social cues that are specific to a user, either in kind or in magnitude, and modify the camera data stream to make subtle social cues more pronounced and exaggerated social cues less pronounced to correspond to some normalized expression. Alternatively, or in addition, the processor 102 may modify the camera data streams to enhance or diminish other social cues according to the user's preference or demonstrated ability to recognize such cues. For example, where a user is especially unobservant of social cues, they may be exaggerated for that user.

In at least one embodiment, the processor 102 transfers the stored camera data and other correlated system and task data to an offline storage device for later analysis and correlation to historic data and other outside factors such as crew rest, crew sleet rhythms, flight schedules, etc. Such transfer may be in real time via the wireless communication device 112. Furthermore, team members may be correlated against each other and other team members for similar tasks to identify teams with complimentary emotional state and social cue patterns.

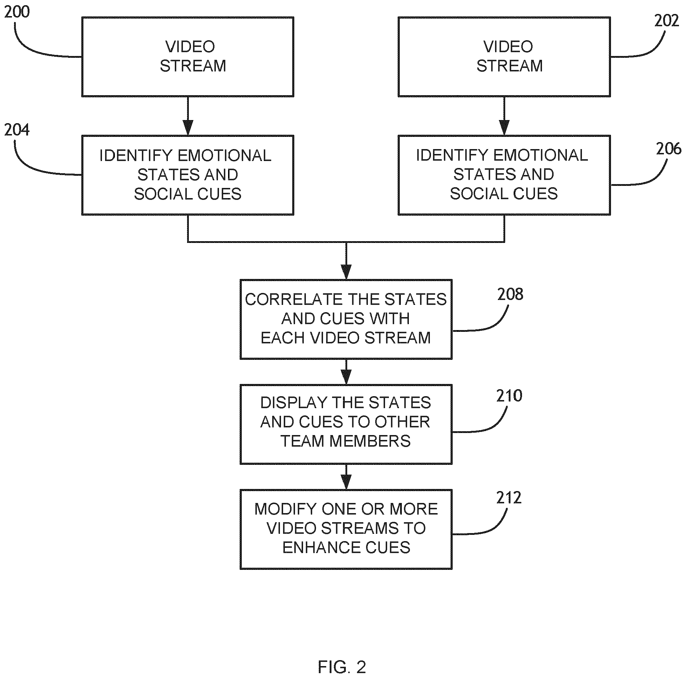

Referring to FIG. 2, a flowchart of an exemplary embodiment of the inventive concepts disclosed herein is shown. Computer systems implementing embodiments of the inventive concepts disclosed herein each receive 200, 202 a video stream corresponding to one or more cameras. The video stream is processed for eye tracking data (including pupil dynamics and eyelid position) to identify 204, 206 emotional states and social cues for a corresponding user. In at least one embodiment, the emotional states and social cues are identified 204, 206 with respect to a user specific profile of facial expressions and idiosyncrasies. Such data is continuously logged. For example, each computer system may compare eye gaze to predetermined expected eye gaze or scan patterns depending on certain stimuli, including facial expressions of other team members as identified 204, 206 by their corresponding systems. Furthermore, the computer system may identify 204, 206 emotional states and social cues by an algorithmic model or machine learning algorithm.

In at least one embodiment, each computer system (or some separate computer system) receives each user emotional states and social cues, and potentially each video stream, and correlates 208 them with respect to each other. Each computer system displays 210 other team member's video streams, and includes indicia of the identified 204, 206 emotional states and social cues.

In at least one embodiment, each computer system may modify 212 each of the video streams according to the identified 204, 206 emotional states and social cues. For example, facial expressions may be enhanced, gaze may be directed toward a speaking, or the like.

In at least one embodiment, the system receives physiological data from one or more physiological sensors such as an EEG, an fNIRs, a heart rate monitor, galvanic skin response, etc. Such physiological data provides the addition metric of neuroactivity when identifying 204, 206 emotional states and social cues. Likewise, the system may receive data related to factors specific to the task. Such task specific data provides the additional metric of context when identifying 204, 206 emotional states and social cues. Such analysis may include processing via machine learning, neural network algorithms.

In at least one embodiment, the system may compile data to facilitate the implementation of one or more of the future actions without the intervention of the user, and potentially before the user has made a determination of what future actions will be performed. The system may prioritize data compilation based on the determined probability of each future action.

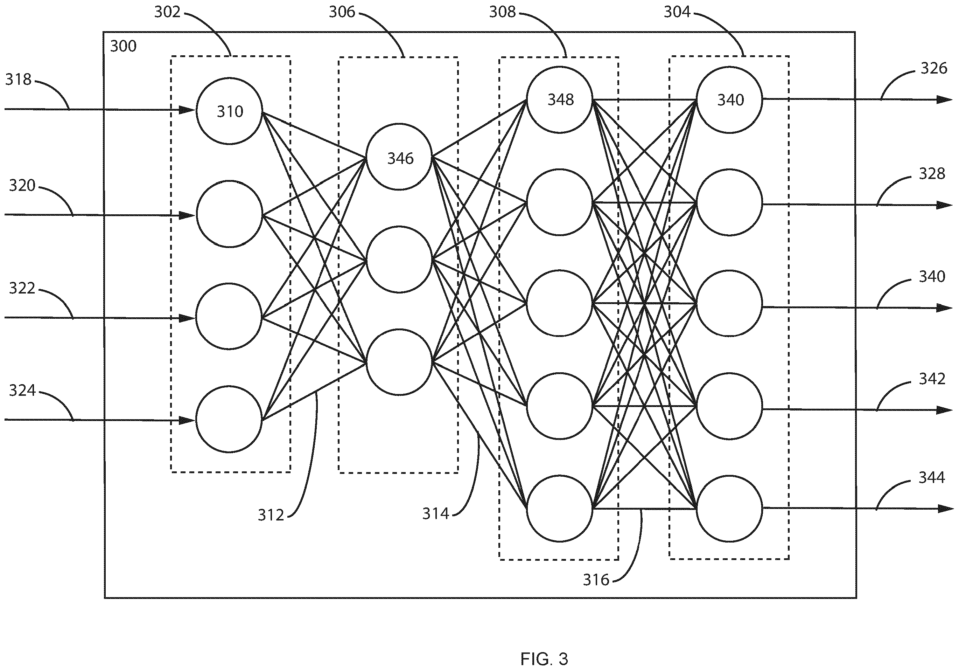

Referring to FIG. 3, a block diagram of a neural network 300 according an exemplary embodiment of the inventive concepts disclosed herein is shown. The neural network 300 comprises an input layer 302 that receives external inputs (including physiological signals, such as EEG, fNIRs, heart rate monitor, galvanic skin response, etc., camera data, and potentially user or task specific profiles), and output layer 304, and a plurality of internal layers 306, 308. Each layer comprises a plurality of neurons or nodes 310, 336, 338, 340. In the input layer 302, each node 310 receives one or more inputs 318, 320, 322, 324 corresponding to a digital signal and produces an output 312 based on an activation function unique to each node 310 in the input layer 302. An activation function may be a Hyperbolic tangent function, a linear output function, and / or a logistic function, or some combination thereof, and different nodes 310, 336, 338, 340 may utilize different types of activation functions. In at least one embodiment, such activation function comprises the sum of each input multiplied by a synaptic weight. The output 312 may comprise a real value with a defined range or a Boolean value if the activation function surpasses a defined threshold. Such ranges and thresholds may be defined during a training process. Furthermore, the synaptic weights are determined during the training process.

Outputs 312 from each of the nodes 310 in the input layer 302 are passed to each node 336 in a first intermediate layer 306. The process continues through any number of intermediate layers 306, 308 with each intermediate layer node 336, 338 having a unique set of synaptic weights corresponding to each input 312, 314 from the previous intermediate layer 306, 308. It is envisioned that certain intermediate layer nodes 336, 338 may produce a real value with a range while other intermediated layer nodes 336, 338 may produce a Boolean value. Furthermore, it is envisioned that certain intermediate layer nodes 336, 338 may utilize a weighted input summation methodology while others utilize a weighted input product methodology. It is further envisioned that synaptic weight may correspond to bit shifting of the corresponding inputs 312, 314, 316.

An output layer 304 including one or more output nodes 340 receives the outputs 316 from each of the nodes 338 in the previous intermediate layer 308. Each output node 340 produces a final output 326, 328, 330, 332, 334 via processing the previous layer inputs 316, the final output 326, 328, 330, 332, 334 corresponding to identified social cues for one or more team members. Such outputs may comprise separate components of an interleaved input signal, bits for delivery to a register, or other digital output based on an input signal and DSP algorithm. In at least one embodiment, multiple nodes may each instantiate a separate neural network 300 to process social cues for a single corresponding team member. Furthermore, the output 326, 328, 330, 332, 334 may include a modification to a video stream to enhance or apply identified social cues. Each neural network 300 may receive data from other team members as inputs 318, 320, 322, 324. Alternatively, a single neural network 300 may receive inputs 318, 320, 322, 324 from all team members, or a separate neural network 300 may receive inputs 318, 320, 322, 324 from each team member's neural network 300 to determine social cues and apply enhancements to one or more video streams.

In at least one embodiment, each node 310, 336, 338, 340 in any layer 302, 306, 308, 304 may include a node weight to boost the output value of that node 310, 336, 338, 340 independent of the weighting applied to the output of that node 310, 336, 338, 340 in subsequent layers 304, 306, 308. It may be appreciated that certain synaptic weights may be zero to effectively isolate a node 310, 336, 338, 340 from an input 312, 314, 316, from one or more nodes 310, 336, 338 in a previous layer, or an initial input 318, 320, 322, 324.

In at least one embodiment, the number of processing layers 302, 304, 306, 308 may be constrained at a design phase based on a desired data throughput rate. Furthermore, multiple processors and multiple processing threads may facilitate simultaneous calculations of nodes 310, 336, 338, 340 within each processing layers 302, 304, 306, 308.

Layers 302, 304, 306, 308 may be organized in a feed forward architecture where nodes 310, 336, 338, 340 only receive inputs from the previous layer 302, 304, 306 and deliver outputs only to the immediately subsequent layer 304, 306, 308, or a recurrent architecture, or some combination thereof.

Embodiments provide a mechanism to understand how remote team members are working together and facilitate communication by enhancing social cues. This can influence team formation and resource allocation, as well as help identify when interventions may be needed to help specific team members.

It is believed that the inventive concepts disclosed herein and many of their attendant advantages will be understood by the foregoing description of embodiments of the inventive concepts, and it will be apparent that various changes may be made in the form, construction, and arrangement of the components thereof without departing from the scope of the claims; and individual features from various embodiments may be combined to arrive at other embodiments.

## Claims

1. A computer apparatus comprising:
at least one camera (108);
a display (114);
a data communication device (112); and
at least one processor (102) in data communication with a memory (104) storing processor executable code,
wherein the processor executable code configures the at least one processor to:
receive a video stream from the at least one camera;
receive a predefined baseline user measurement of at least gaze and pupil dynamics, and personal facial expression idiosyncrasies;
determine one or more emotional states and social cues based on the video stream compared to the predefined baseline;
receive one or more contemporaneous team member emotional states and social cues via the data communication device;
send the one or more emotional states and social cues to each team member; and
display the one or more contemporaneous team member emotional states and social cues associated with a corresponding team member.

2. The computer apparatus of Claim 1, further comprising one or more physiological data recording devices in data communication with the at least one processor, wherein:
the processor executable code further configures the at least one processor to:
receive physiological data from the one or more physiological data recording devices; and
correlate the physiological data with the video stream; and
identifying the one or more emotional states and social cues includes reference to the physiological data.

3. The computer apparatus of Claim 2, wherein:
the processor executable code further configures the at least one processor to receive a task or user specific profile of gaze, scan pattern, facial expression, and physiological data; and
identifying the one or more emotional states and social cues includes reference to the task or user specific profile.

4. The computer apparatus of any preceding Claim, wherein the processor executable code further configures the at least one processor to:
determine a relative gaze direction with respect a speaking team member; and
modify one or more video streams to render a team members gaze toward the speaking team member.

5. The computer apparatus of any preceding Claim, wherein the processor executable code further configures the at least one processor to:
determine a modification to a video stream to enhance at least one of the one or more emotional states and social cues in a corresponding video stream; and
apply the modification to the corresponding video stream.

6. The computer apparatus of any preceding Claim, wherein the processor executable code further configures the at least one processor as a machine learning neural network.

7. A method comprising:
receiving a video stream from at least one camera (108);
receiving a predefined baseline user measurement of at least gaze and pupil dynamics, and personal facial expression idiosyncrasies;
determining one or more emotional states and social cues based on the video stream compared to the predefined baseline;
receiving one or more contemporaneous team member emotional states and social cues;
sending the one or more emotional states and social cues to each team member; and
displaying the one or more contemporaneous team member emotional states and social cues associated with a corresponding team member.

8. The method of Claim 7, further comprising:
receiving physiological data from one or more physiological data recording devices; and
correlating the physiological data with the video stream,
wherein identifying the one or more emotional states and social cues includes reference to the physiological data.

9. The method of Claim 8, further comprising receiving a task or user specific profile of gaze, scan pattern, facial expressions, and physiological data, wherein identifying the one or more emotional states and social cues includes reference to the task or user specific profile.

10. The method of Claim 7, 8 or 9, further comprising:
determining a relative gaze direction with respect a speaking team member; and
modifying one or more video streams to render a team members gaze toward the speaking team member.

11. The method of Claim 7, 8, 9 or 10, further comprising:
determining a modification to a video stream to enhance at least one of the one or more emotional states and social cues in a corresponding video stream; and
applying the modification to the corresponding video stream.

12. The method of any of Claims 7 to 11, further comprising:
receiving at least one video stream from a team member;
displaying the at least one video stream from the team member on a display; and
determining the user emotional states and social cues with reference to the at least one video stream from the team member.

13. The method of any of Claims 7 to 12, further comprising:
recording the one or more emotional states and social cues, and one or more contemporaneous team member emotional states and social cues over time; and
determining a team composition based on one or more emotional states and social cues, and one or more contemporaneous team member emotional states and social cues.

14. A team monitoring system comprising:
a plurality of team member monitoring computers, each comprising a computer apparatus as claimed in any of claims 1 to 6.
